Europäisches Patentamt

European Patent Office

Office européen des brevets

(11) Publication number: **0 499 330 A1**

## EUROPEAN PATENT APPLICATION

(21) Application number: **92200390.0**

(22) Date of filing: **11.02.92**

(51) Int. Cl.5: **A61B 10/00**

(30) Priority: **13.02.91 NL 9100246**

(43) Date of publication of application:
**19.08.92 Bulletin 92/34**

(84) Designated Contracting States:
**AT BE CH DE DK ES FR GB GR IT LI LU MC NL PT SE**

(71) Applicant: **ROVERS B.V.**
**Lekstraat 10**
**NL-5347 KV Oss(NL)**

(72) Inventor: **Rovers, Willem Pieter Gerard**
**Lekstraat 12**
**NL-5347 KV Oss(NL)**

(74) Representative: **Metman, Karel Johannes et al**
**Octrooibureau Los en Stigter B.V. P.O.Box 20052**
**NL-1000 HB Amsterdam(NL)**

(54) **Device for collecting cells from a body cavity.**

(57) A device for collecting cells from a body cavity comprises an elongate stem (1) and a head (2) formed on a free end of the stem. Said head has a carrier (5) extending substantially axially of the device, and cell removing elements (7, 8) distributed circumferentially and longitudinally around the carrier. Said carrier and said cell removing elements are integrally formed from flexible plastic material. Said cell removing elements include alternating axial rows of teeth (7) having scraping edges (9) and axial ribs (8) having chaffing edges (10).

fig.1

EP 0 499 330 A1

The invention relates to a device for collecting cells from a body cavity, comprising an elongate holding means and, on a free end thereof, a head having a carrier extending substantially axially and carrying cell removing means distributed along the circumference and length thereof.

In a presently used embodiment of such a device for obtaining a smear sample from the cervix, which is known as the "Cytobrush", the carrier consists of steel wires to which bristles as cell removing means are attached radially. This device has the disadvantage that only a small amount of cells can be sampled as a result of the fact that when the head of the device is removed from the cervical canal the cells that are collected on the bristles are given back again to the wall of the canal. As a consequence, the chances of obtaining a representative smear sample are adversely affected.

Us patents 2,514,665; 3,540,432; 3,774,590; and 4,127,113 disclose further devices for obtaining smear samples from the uterus mouth and the cervical canal, which did not lead to a success in practice due to various shortcomings.

In the present practice of collecting cells from the urethra it is common to use a small stick or wire having a small tip of cotton thereon. The disadvantage thereof is that the humid cells are absorbed into the cotton and are not delivered again easily. Furthermore the tip sometimes gets stuck wholly or partially within the urethra where it may lead to inflammation and in certain cases it can only be removed through surgery.

It is an object of the invention to provide a device of the type mentioned in the preamble, with which a large number of cells can be collected from a body cavity in a reliable manner.

For this purpose the device according to the invention is characterized in that the carrier and cell removing means are made integrally from flexible plastic material, and the cell removing means include teeth having scraping edges.

Due to the flexibility of the carrier, the device according to the invention has the advantage that it is able to conform to the configuration of the body cavity and thereby avoid trauma on the one hand and is able to come properly and uniformly into contact with the wall of the body cavity on the other hand. The cell removing means constructed as teeth are very well capable of removing cells from the wall of the body cavity, while these cells are allowed to gather at the base of the teeth and to remain there, protected by the teeth, when the device is removed from the body cavity. As a result a large number of cells may be collected in a reliable manner, which promotes the representativity of the sample.

Preferably the scraping edges of the teeth extend substantially axially, wherein the teeth may be arranged on the carrier in axially directed rows. Consequently cells are only collected when the device is rotated about the axis.

It is favourable when the cell removing means also include ribs extending substantially axially and, preferably, the teeth and ribs being arranged alternately in circumferential direction of the carrier.

In this embodiment the ribs mainly have a chaffing function to collect primarily liquid cells and cells that are released from the wall but not carried along by the leading teeth which rather scrape than chafe. This combination of teeth and ribs leads to an optimization with regard to the number of cells that may be collected in a reliable and painless manner.

The invention will hereafter be elucidated with reference to the drawing showing various embodiments of the invention by way of example.

Fig. 1 is an enlarged side view, partially in section, of a device for collecting cells from the cervical canal of the uterus of a female in accordance with the present invention.

Fig. 2 and 3 are slightly enlarged transverse sectional views along the lines II-II and III-III, respectively, of Fig. 1.

Fig. 4 is a view, corresponding to that of Fig. 1, of a device for collecting cells from the urethra of a male or female in accordance with the present invention.

Fig. 5 and 6 are slightly enlarged transverse sectional views along the lines V-V and VI-VI, respectively, of Fig. 4.

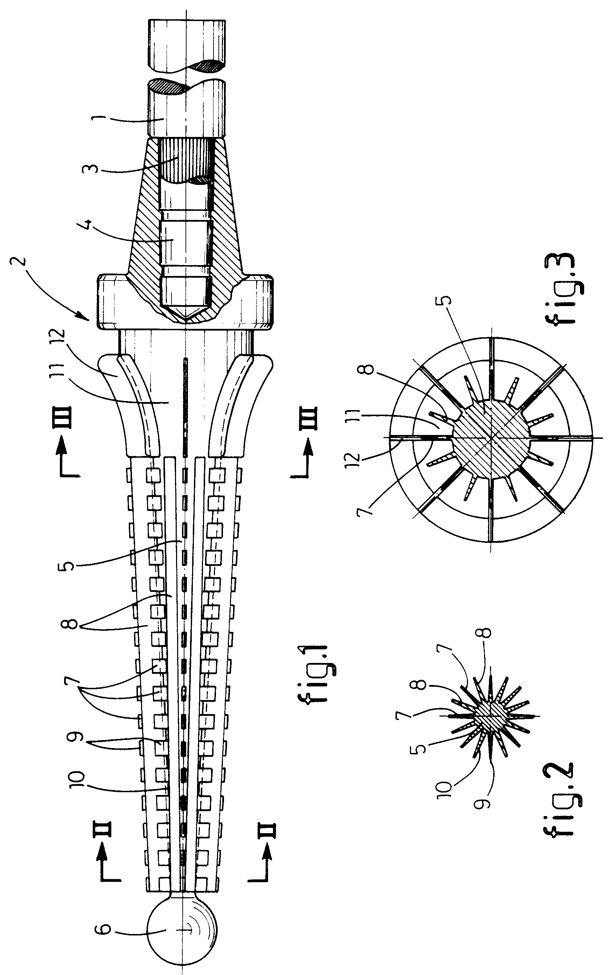

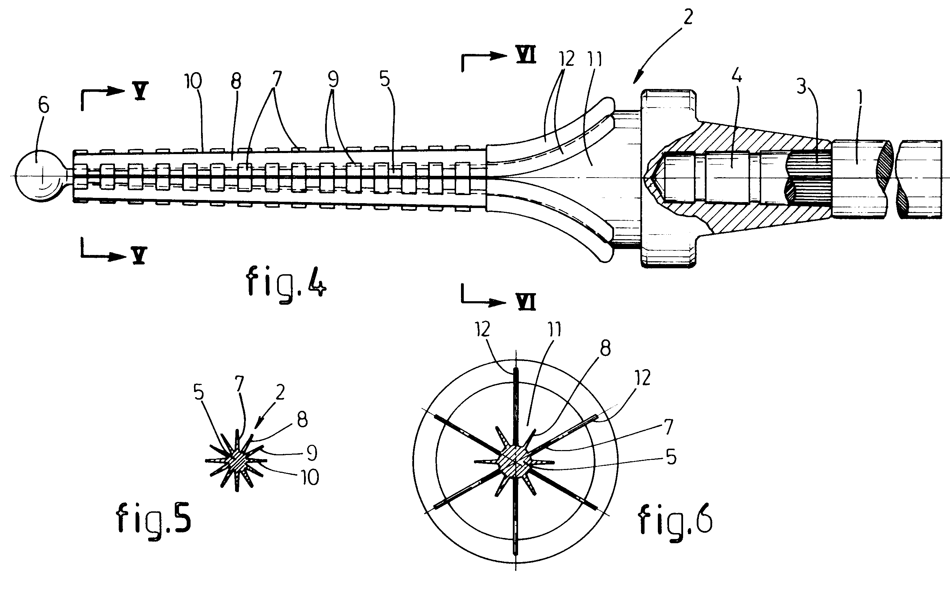

The drawing shows embodiments of the device for collecting cells from a body cavity, wherein the embodiment of Fig. 1-3 is particularly suited for obtaining a smear sample of the cervical canal of the uterus of a female, and the embodiment of Fig. 4-6 is suited for obtaining a smear sample from the urethra of a male or female. The device according to the invention is intended to discover all kinds of viruses and bacterias, such as HIV (aids), HPV (carcinoma), HSV, chlamydia and the like. The invention, however, may also be used in colonoscopy for taking cell samples from the gastrointestinal tract.

In the embodiment of Fig. 1-3 the device includes an elongate holding means, such as a stem 1, to which a head 2 is removably attached by a pin 3 on one end of the stem 1, which is tightly inserted into a bore 4 in the head 2 made of elastic material.

The head 2 of the device includes a core or axial carrier 5 having a substantially circular cross section. The carrier 5 slightly tapers in distal direction and has its distal end provided with a round head 6 to prevent trauma when the head 2 is inserted into the body cavity.

Regularly spaced about the circumference of the carrier 5 are a plurality of axially extending rows of teeth 7 which are alternated with longitudinal ribs 8. Both the rows of teeth 7 and the ribs 8 extend substantially along the whole length of the carrier 5. The teeth 7 are positioned substantially radially on the carrier 5 and have scraping edges 9 on their radial outer side, which extend mainly axially with respect to the carrier 5 and thereby follow the taper of the carrier 5. The ribs 8 are also positioned radially on the carrier 5 and each having a chaffing edge 10 on their radial outer side. In this embodiment by way of example the teeth 7 and ribs 8 have the same radial extent in each cross section of the carrier 5.

At the proximal end of the carrier 5 is a portion 11 widening from the carrier 5 and having arranged thereon further ribs 12 in extension of the rows of teeth 7. This thicker portion 11 serves on the one hand as a stop for limiting the depth of penetration of the head 2 into the cervical canal, while on the other hand the ribs 12 disposed thereon may collect cells at the entrance to the cervical canal.

The head 2 is an integral part of flexible plastic material which may consist of polyethylene, thermoplastic rubber and silicon rubber, for example. The head 2 is preferably made by injection moulding.

The operation of the device is based on that, after the carrier 5 of the head 2 is inserted into the cervical canal, it is manually rotated about the axis through the stem 1. In the present embodiment the teeth 7 and ribs 8 are formed symmetrically on the carrier 5, so that this rotation may be to the left or to the right, but other embodiments are feasible in which the rotation may take place in only one direction. When the carrier 5 is rotated about its axis the teeth 5 will exert a scraping action onto the wall of the canal whereby solid material is removed from the wall. Contrary thereto the ribs 8 will exert more of a chaffing action whereby only liquid material is taken from the wall as well as any scales or the like loosened by the teeth 7. The alternate arrangement of the teeth 7 and ribs 8 in combination with the large number of teeth 7 and ribs 8 (in this embodiment by way of example eight rows of teeth 7 and eight ribs 8) make it possible to remove a very large number of cells from the tissue wall. Due to the flexibility of the teeth 7 and ribs 8 it is even so allowed to effect a painless sampling operation. The chafed and scraped cells are collected within the spaces at the base of the teeth 7 and ribs 8 and when the head 2 of the device is removed from the canal the cells collected there remain in their position and are not delivered back to the cellular wall since the rigidity of the teeth 7 and ribs 8 in axial direction is large and when the head 2 is removed from the canal the cells thereon are prevented from coming into contact with the wall of the canal. In this manner a large number of cells is actually obtained in a sample so that a very good representativity of the sample can be obtained. The flexibility of the teeth 7 and ribs 8 then allows that the collected cells are easily delivered to a microscopic slide by turning the carrier 5 with its teeth and ribs along the slide. Of course it is also possible to transport the head 2 separated from the stem 1 within a preservation liquid to the laboratory in order to have the sample examined there. Tests have proven that with the device according to the invention a sample containing 400.000 - 500.000 cells may easily be collected, which is nearly ten times as much as presently obtained with the so called "Cytobrush".

Fig. 4-6 show an embodiment of the device for collecting cells from the urethra which is, to a large extent, similar to the embodiment of Fig. 1-3. Only the diameter of the carrier 5 having the teeth 7 and ribs 8 arranged thereon is smaller, while also a smaller number of rows of teeth 7 and ribs 8 is provided.

In the embodiment of Fig. 4-6 the diameter of the round head 6 could be 2.8 mm, the carrier 5 tapering 3° over a length of 23.9 mm. The radial height of the teeth and ribs 8 is 1 mm and the axial length of the teeth 7 is 0.8 mm. In the embodiment of Fig. 1-3, the round head 6 could have a diameter of 3.5 mm and the taper of the carrier 5 being also 3° over a length of 23.9 mm. The teeth and ribs heigth is 1.2 mm and tooth length is 0.8 mm.

The invention is not restricted to the embodiments described herein before and shown in the drawing by way of example, which may be varied in different manners within the scope of the invention. It would for instance be possible to provide only ribs or only teeth on the carrier, wherein several patterns are feasible. It is possible, for example, to arrange a plurality of staggered axial rows of teeth. When both teeth and ribs are provided the radial extent of the teeth and ribs may differ in the same cross section. Furthermore the carrier may be truly cylindrical rather than tapering, depending on the particular use. Also other cross sections of the carrier instead of round are conceivable, but a round cross section having a large number of cell removing means is most favourable with regard to the pressure on the wall of the body cavity and the number of cells to be collected.

**Claims**

1. A device for collecting cells from a body cavity, comprising
   - an elongate holding means (1); and
   - a head (2) formed on a free end of the holding means (4), said head having a carrier (5) extending substantially axially of the device, and cell removing means (7, 8) distributed circumferentially and longitudinally around the carrier (5), wherein said carrier (5) and said cell removing means (7, 8) are integrally formed from flexible plastic material, and said cell removing means (7, 8) include teeth (7) having scraping edges (9).

2.  A device according to claim 1, wherein the scraping edges (9) of the teeth (7) extend substantially axially.

3.  A device according to claim 2, wherein the teeth (7) are provided on the carrier (5) in axially directed rows.

4.  A device according to one of claims 1-3, wherein the cell removing means (7, 8) also include ribs (8) having chaffing edges (10) and extending substantially axially.

5.  A device according to claims 3 and 4, wherein the teeth (7) and ribs (8) are arranged alternately in circumferential direction of the carrier (5).

6.  A device according to one of claims 1-5, wherein the cell removing means (7, 8) are positioned substantially radially on the carrier (5).

7.  A device according to one of claims 1-6, wherein the carrier (5) has a proximal end connecting to a widening portion (11) carrying ribs (12) formed on the head (2).

8.  A device according to one of claims 1-7, wherein said flexible plastic material for the carrier (5) and cell removing means (7, 8) is chosen from the group of polyethylene, thermoplastic rubber and silicon rubber.

fig.1

fig.2

fig.3

EP 0 499 330 A1

fig.4

fig.5

fig.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CH-A-653 543 (FOPPIANO) <br> * page 2, column 2, line 3 - line 13 * <br> --- | 1-3,6,8 | A61B10/00 |
| A | US-A-4 981 143 (SAKITA ET AL) <br> * column 4, line 9; figure 8 * <br> --- | 2 | |
| A | EP-A-0 262 966 (ANIMAL HOUSE) <br> * page 3, line 39 - line 50; figure 1 * <br> --- | 4 | |
| A | US-A-2 839 051 (CHESTER) <br> --- | | |
| P,X | WO-A-9 116 004 (MOHAJER) <br> * abstract; figure 3 * <br> --- | 1 | |
| P,X | EP-A-0 448 137 (FUTUREROLE) <br> * abstract; figure 2 * | 1 | |
| | ----- | | TECHNICAL FIELDS SEARCHED (Int. Cl.5) |
| | | | A61B |

The present search report has been drawn up for all claims

| Place of search | Date of completion of the search | Examiner |
|---|---|---|
| THE HAGUE | 24 APRIL 1992 | BARTON S. |

EPO FORM 1503 03.82 (P0401)